# EUROPEAN PATENT SPECIFICATION

(11) **EP 2 533 800 B1**
(45) Date of publication and mention of the grant of the patent: **20.07.2016**
(21) Application number: 11741984.6
(22) Date of filing: 11.02.2011
(51) Int. Cl.: A61K 38/24, A61K 9/00

(54) **LIQUID FORMULATION OF FOLLICLE STIMULATING HORMONE**
FLÜSSIGE FORMULIERUNG AUS EINEM FOLLIKELSTIMULIERENDEN HORMON
FORMULATION LIQUIDE DE L'HORMONE DE STIMULATION FOLLICULAIRE

(30) Priority: 12.02.2010 IN MU03842010
(43) Date of publication of application: 19.12.2012
(73) Proprietor: Intas Pharmaceuticals Ltd., Ahmedabad, Gujarat 380009 (IN)
(72) Inventor: MAJUMDER, Sudip Kumar, West Bengal (IN); SHARMA, Manish Kumar, Maharashtra (IN); GUPTA, Tarun Kumar, Uttar Pradesh (IN)
(74) Representative: Santamaria Gamez, Antoni
(86) International application number: PCT/IN2011/000090
(87) International publication number: WO 2011/099036

(56) References cited:
- EP-A2- 0 974 359
- WO-A1-2007/037607
- WO-A1-2009/056569
- WANG.: 'Instability, stabilization, and formulation of liquid protein pharmaceuticals.' INT J PHARM vol. 185, no. 2, 1999, pages 129 - 188, XP002323952

## Description

### FIELD OF THE INVENTION

The present invention relates to a novel aqueous formulation of human follicle stimulating hormone (FSH) which is stable for a prolonged period of time at ambient or body temperature to guarantee a reasonable shelf-life. More specifically, it relates to an aqueous formulation of human follicle stimulating hormone comprising a therapeutically effective amount of human FSH stabilized in a buffer containing one or more stabilizer, a non-ionic surfactant, an anti-oxidant, inorganic salt, and one or more preservatives as defined in the claims.

### BACKGROUND OF THE INVENTION

A human follicle stimulating hormone (FSH) is a hormone which plays an important role in the reproductive functions, in conjunction with Luteinizing Hormone (LH) and human Chorionic Gonadotropin (hCG). Structurally, FSH is a heterodimeric glycoprotein formed by the non-covalent association of alpha and beta subunits. Alpha subunit consists of 92 amino acid residues while beta subunit consists of 111 amino acid residues, each of which retains two Asn-linked glycosylation sites.

Until the 1980s, the primary source of FSH was urine of pregnant women. A further purified form of urine-derived FSH was introduced in the 1990s, and later a recombinant FSH was developed and widely used since 1998. With the advent of recombinant DNA technology, it became possible to produce human FSH in cell cultures transfected with the nucleic acid sequences coding for the alpha and beta chain. DNA sequences coding for the alpha and beta chains, and methods for producing recombinant FSH have been disclosed in WO88/10270, WO86/04589 and EP0735139. FSH stimulates the growth and maturation of the ovarian follicles in females. Therefore, FSH is a critical hormone responsible for ovulation of females and is used in the infertility treatment of anovulatory infertile women.

Currently, in the market, there are four commercial recombinant human FSH products; Gonal - F^{®} and Gonal-F RFF^{®} from Merck Serono, and Puregon^{®} and Follistim AQ^{®} from Organon (Schering Plough), which are produced in Chinese Hamster Ovary (CHO) cells by recombinant DNA technology.

Generally, proteins have a very short half-life, and undergo denaturation (such as aggregation, dissociation, and adsorption on the surface of vessels) upon exposure to various factors such as unfavorable temperatures, water-air interface, high pressure, physical/ mechanical stress, organic solvents and microbial contamination. Consequently, the denatured protein loses intrinsic physicochemical properties and physiological activity. Denaturation of proteins is often irreversible and therefore proteins, once denatured, may not recover their native properties to the initial state.

In particular, proteins, which have a heterodimeric structure consisting of two different subunits such as FSH, have a tendency to dissociate in aqueous solutions and to adsorb on the inner surface of vessels. The dissociated proteins lose their own physiological activity, and the dissociated monomers are readily susceptible to dissociation and aggregation. Further, the proteins adsorbed on the inner surface of vessels are vulnerable to aggregation via the denaturation process. When they are administered into the human body, the aggregated proteins may serve as the cause of formation of antibodies against an injected protein and the naturally occurring protein in the human body as well. Therefore, the proteins of interest should be administered in a sufficiently stable state. To this end, a great deal of research has been made on the development of a method for preventing denaturation of proteins in the solution.

To overcome the stability problem of proteins, some of the protein-based drug products are made more stable via lyophilization (freeze-drying). As an example, US5270057 discloses a lyophilized formulation comprising gonadotropin stabilized with polycarboxylic acid or a salt thereof, preferably citric acid and a non-reducing disaccharide, sucrose. US5650390 discloses a lyophilized formulation comprising FSH, LH or hCG stabilized by means of a combination of sucrose and glycine. However, lyophilized products are inconvenient in that they have to be dissolved in Water for Injection (WFI) for reconstitution prior to their use. Further, the production process of the lyophilized products involves a freeze-drying step which could result in freeze-injury to the protein lyophilized.

US5929028 discloses a stable liquid formulation of gonadotropin, which is prepared by dissolving gonadotropin [e.g. LH, TSH (Thyroid Stimulating Hormone), FSH and hCG], using a diluent composed of a stabilizing amount of polycarboxylic acid or a salt thereof, a stabilizing amount of a thioether compound, a non-reducing disaccharide like sucrose and a non-ionic surfactant. According to this art, sodium citrate is described as the most preferred form of polycarboxylic acid or a salt thereof and the formulation contains 1.47% sodium citrate. This composition should be stored refrigerated at 2-8 °C until dispensed. Upon dispensing, the product may be stored by the patient at 2-8°C until the expiration date, or at or below 25°C (77°F) for three months, whichever occurs first.

EP1610822 application describes the pharmaceutical formulations of FSH, LH, and mixtures of FSH and LH, and methods of producing such formulations. The invention describes a liquid or freeze-dried formulation of FSH or LH or FSH and LH comprising a surfactant selected from Pluronic^{®}F77, Pluronic^{®}F87, Pluronic^{®}F88, and Pluronic^{®}F68. The composition as described in this patent application requires refrigeration at 2-8°C until dispensed. Upon reconstitution with the diluent, the Pen device containing the drug product may be stored by the patient at 2-8°C until the expiration date or at room temperature 20-25 ° C for up to three months or until the expiration date, whichever occurs first.

WO2007/037607 discloses an aqueous formulation of FSH which is stabilized to maintain the activity of FSH for a prolonged period of time. The patent application claims an aqueous formulation comprising a therapeutically effective amount of FSH stabilized in a phosphate buffer containing glycine, methionine and a non-ionic surfactant, preferably polysorbate 20, which is capable of maintaining the activity of FSH for an extended period of time. The composition contains glycine, methionine, polysorbate 20 as stabilizer and 90% pure FSH which is stable at room temperature upto six months and undergoes less oxidation.

WO2009/098318 application discloses a liquid pharmaceutical composition comprising a FSH polypeptide and benzalkonium chloride and benzyl alcohol as preservatives. The composition further comprises optionally one or more additional pharmaceutically acceptable excipients. In one embodiment, the composition contains methionine as an antioxidant. The composition shows good storage stability when stored at 2-8°C. This FSH aqueous formulation comprising of polysorbate 20 as a surfactant, mannitol as a tonicity modifier, phosphate as a buffer, methionine as a stabilizing agent, and benzyl alcohol and benzalkonium chloride as preservatives prevents protein loss as well as dissociation of the protein into constituent monomers, thereby, stabilizing FSH for a prolonged period of time.

It can be seen from the above arts that the composition of FSH has to be stored refrigerated at
2-8 ° C until dispensed. Upon dispensing, the product may be stored by the patient at 2-8°C until the expiration date or below 25°C (77°F) for three months, whichever occurs first. Therefore, there is a need for the development of a novel, thermostable, aqueous formulation of FSH which is stabilized to maintain its activity even at elevated temperatures (e.g. body temperature) as FSH is self-administered and therefore, would be convenient to carry while traveling or during storage, obviating the need to maintain the product in refrigerated (2-8°C) conditions.

The present invention has been made in view of the problems faced by end-user who may need to carry the product in a liquid form at room temperature, ambient temperature or at body temperature without the need of refrigeration for a prolonged period of time to guarantee a reasonable shelf-life.

### SUMMARY OF THE INVENTION

The present invention provides a novel aqueous formulation of human follicle stimulating hormone (FSH) which is stable for a prolonged period of time at ambient or body temperature to thus maintaining a reasonable shelf-life.

In one aspect the invention provides an aqueous formulation of human FSH comprising a therapeutically effective amount of human FSH stabilized in a buffer system containing one or more stabilizer, a non-ionic surfactant, an anti-oxidant, inorganic salt, and one or more preservatives as defined in the claims.

In another aspect, the disclosure provides a formulation of human FSH or its variant and an inorganic salt selected from the group consisting of sodium chloride, calcium chloride and potassium chloride which acts as a thermostable agent.

In yet another aspect, of the disclosure the formulation contains a polyol which is sucrose, trehalose, maltose, mannitol, sorbitol, maltitol or xylitol, either alone or in combination thereof.

The human FSH formulation of the disclosure also consists of a polysorbate-based non-ionic surfactant or a poloxamer-based non-ionic surfactant or a combination thereof.

In one aspect, of the disclosure the novel aqueous formulation of human FSH also contains antioxidant selected from the group consisting of L-methionine, sodium bisulfite, salts of ethylenediamine tetraacetic acid (EDTA), butylated hydroxytoluene (BHT) and butylated hydroxyl anisole (BAH).

The disclosure also provides for the addition of one or more preservative to the human FSH formulation selected from the group consisting of phenol, m-cresol, p-cresol, o-cresol, chlorocresol, alkylparaben, benzethonium chloride, sodium dehydroacetate and thimerosal or a combination thereof.

The novel aqueous human FSH formulation of the disclosure is maintained at a pH of about 6.5 to 7.2 in a buffer system selected from the group buffers consisting of phosphate, succinate, acetate, citrate, arginine, TRIS and histidine or a combination thereof.

The present invention provides a formulation of human FSH comprising sodium chloride, trehalose, mannitol, polysorbate 20, L-methionine, phenol, monobasic sodium phosphate monohydrate and dibasic sodium phosphate dihydrate and maintained at a pH of about 6.5 to 7.2.

The present invention also provides a formulation of human FSH comprising sucrose, L-methionine, polysorbate 20, phenol, monobasic sodium phosphate monohydrate and dibasic sodium phosphate dehydrate and maintained at a pH of about 6.5 to 7.2

### BRIEF DESCRIPTION OF THE DRAWINGS

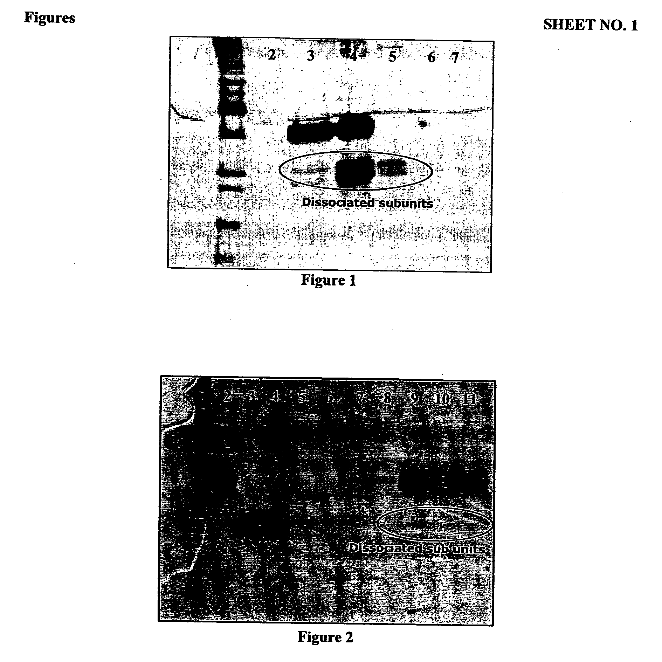

**Figure 1** shows the comparison of the dissociation of alpha and beta subunits of recombinant human follicle stimulating hormone composition kept at 40°C for 7 days
   **Lane 1:** Prestained Marker
   **Lane 2:** Sample buffer
   **Lane 3:** Composition given in Table 1 (protein loaded 4.4 ug)
   **Lane 4:** Reference Medicinal Product (Gonal F RFF - Pen) (protein loaded 4.4 ug)
   **Lane 5:** 0.22 µg dissociated (by boiling) of FSH (5.0% of 4.4 µg)
   **Lane 6:** 0.11 µg Intact FSH (2.5% of 4.4µg)
   **Lane 7:** Sample Buffer
**Figure 2** shows the comparison of the dissociation of alpha and beta subunits of recombinant human follicle stimulating hormone composition kept at 2-8°C for 3 months
   **Lane 1:** Prestained Marker
   **Lane 2:** Composition given in Table 1 (protein loaded 2.2 µg)
   **Lane 3:** 0.44 µg dissociated (by boiling) of FSH (10.0% of 4.4 µg)
   **Lane 4:** 0.22 µg dissociated (by boiling) of FSH (5.0% of 4.4 µg)
   **Lane 5:** 0.11 µg dissociated (by boiling) of FSH (2.5% of 4.4 µg)
   **Lane 6:** 0.04 µg dissociated (by boiling) of FSH (1.0% of 4.4 µg)
   **Lane 7:** 0.02 µg Intact FSH (0.5% of 4.4µg)
   **Lane 8:** 0.04 µg Intact FSH (1.0% of 4.4µg)
   **Lane 9:** Composition given in Table 1 (protein loaded 4.4 µg)
   **Lane 10:** Composition given in Table 1 (protein loaded 4.4 µg)
   **Lane 11:** Composition given in Table 1 (protein loaded 4.4 µg)

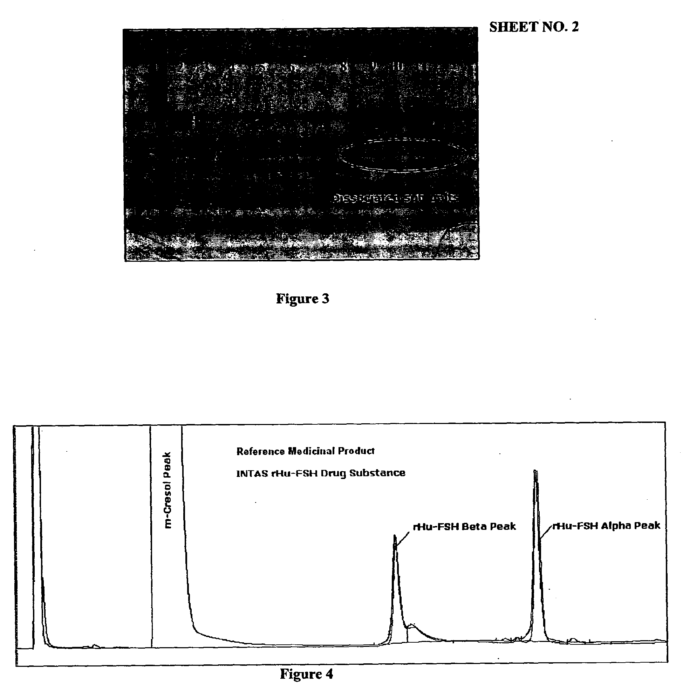

**Figure 3** shows the comparison of the dissociation of alpha and beta subunits of recombinant human follicle stimulating hormone composition kept at 25°C for 3 months
   **Lane 1:** Prestained Marker
   **Lane 2:** Composition given in Table 1 (protein loaded 2.2 µg)
   **Lane 3:** 0.44 µg dissociated (by boiling) of FSH (10.0% of 4.4 µg)
   **Lane 4:** 0.22 µg dissociated (by boiling) of FSH (5.0% of 4.4 µg)
   **Lane 5:** 0.11 µg dissociated (by boiling) of FSH (2.5% of 4.4 µg)
   **Lane 6:** 0.04 µg dissociated (by boiling) of FSH (1.0% of 4.4 µg)
   **Lane 7:** 0.04 µg Intact FSH (1.0% of 4.4µg)
   **Lane 8:** 0.02 µg Intact FSH (0.5% of 4.4µg)
   **Lane 9:** Composition given in Table 1 (protein loaded 4.4 µg)
   **Lane 10:** Composition given in Table 1 (protein loaded 4.4 µg)
   **Lane 11:** Composition given in Table 1 (protein loaded 4.4 µg)
**Figure 4** shows the comparative RP-HPLC profile of composition given in Table 1 with Reference Medicinal Product

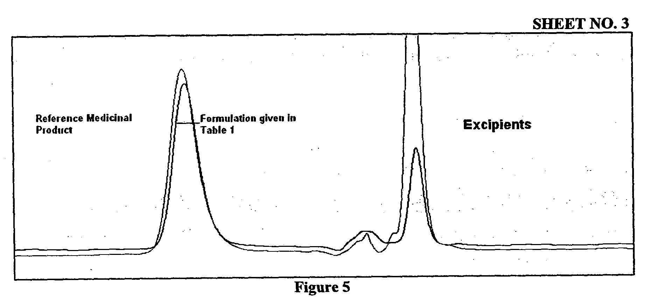

**Figure 5** shows the comparative SE-HPLC profile of composition given in Table 1 (Red line) with Reference Medicinal Product (Blue line)

### DESCRIPTION OF THE INVENTION

The present invention relates to a novel, aqueous formulation of FSH which is stabilized to maintain its activity at elevated temperature with a dissociation of less than 5% after two hours at 55°C.

In one embodiment, the aqueous formulation of the present invention comprises recombinant human follicle stimulating hormone along with suitable buffers, antioxidants, non-ionic surfactants, one or more stabilizers, inorganic salts and optionally one or more preservative.

Human Follicle stimulating hormone (FSH) is produced by recombinant DNA technology. FSH being a complex heterodimeric protein, a eukaryotic cell line has been selected for expression work (Chinese hamster ovary cells). The pharmaceutical preparation of recombinant human FSH (rFSH) differs from that of human menopausal gonadotropin (hMG) and the first generation of urinary FSH (uFSH) in terms of source of bulk protein, purity, specific activity, batch to batch consistency and complete absence of luteinzing hormone activity.

Buffers are used for maintaining pH of the formulation. In another embodiment, of the disclosure the buffer system which is used in the present formulation comprises phosphate buffer, succinate buffer, acetate buffer, citrate buffer, arginine, TRIS buffer, histidine either alone or in suitable combination giving desired pH range from 6.5 to 8. Preferably the buffer used is phosphate buffer either alone or in combination.

Antioxidant, as used herein, refers to a chemical that reduces the quantity of oxidized FSH impurity/ species within a solution containing FSH. The anti-oxidant used in the present disclosure is selected from the group consisting of L-methionine, sodium bisulfite, salts of ethylenediamine tetraacetic acid (EDTA), butylated hydroxytoluene (BHT), and butylated hydroxyl anisole (BAH). More preferably the anti-oxidant is L-methionine. Non-ionic surfactant is used in the present invention in order to prevent adsorption of FSH or FSH variant on the surface of the vial, ampoule, carpoule, cartridge or syringe.

Non-ionic surfactants lower surface tension of a protein solution, thereby preventing its adsorption or aggregation of proteins on to a hydrophobic surface. Preferred examples of non-ionic surfactant that is used in the present disclosure may include a polysorbate-based non-ionic surfactant and a poloxamer based non-ionic surfactant, either alone or in combination.

Stabilizers used in the present disclosure is selected from the group consisting of amino acids such as glycine and alanine, either alone or in combination thereof, monosaccharide such as glucose, fructose, xylose and mannose, either alone or in combination, polyols such as sucrose, trehalose, maltose, mannitol, sorbitol, maltitol and xylitol, either alone or in combination thereof. The presence of polyols protects the molecules during storage at relatively high temperature.

Preservatives refer to excipient or substance added to a formulation to act as a bacteriostatic agent when filled as a multidose. A preserved FSH or FSH variant containing formulation of the present invention preferably meets statutory or regulatory guidelines for preservative effectiveness to be a commercially viable multi-dose product, preferably in humans. Preservatives used in the present disclosure are selected from the group consisting of phenol, m-cresol, p-cresol, o-cresol, chlorocresol, alkylparaben (methyl, ethyl, propyl, butyl and so on), benzethonium chloride, sodium dehydroacetate and thimerosal, either alone or in combination thereof.

Inorganic salts of the disclosure include but not restricted to sodium chloride, calcium chloride and potassium chloride. These salts act as thermostable agents by increasing the melting temperature of the product and also reduce non-specific interaction of the molecule, thereby increasing the stability of the product at higher temperatures. They also prevent the dissociation of the intact FSH into alpha and beta subunits.

The following examples illustrate the pharmaceutical compositions described in the present invention and the means of carrying out the invention to obtain a thermally stable pharmaceutical formulation of human FSH. These examples should not, however, be construed as limiting the scope of the invention.

### Example 1

### Production of recombinant human FSH by recombinant DNA technology

Recombinant human FSH is produced in transfected CHO host cells by standard methods.

### Example 2

### Preparation of hFSH aqueous formulation with trehalose & mannitol

The formulation buffer containing monosodium dihydrogen phosphate monohydrate and disodium hydrogen phosphate dehydrate to prepare phosphate buffer of pH 7.0. Mannitol, trehalose and sodium chloride are added to the phosphate buffer as a tonicity modifier and stabilizer. L-Methionine is added to the solution as an antioxidant and polysorbate 20 as a stabilizer to form a formulation buffer, pH is adjusted with phosphoric acid solution or sodium hydroxide solution to obtain the pH of 7.0±0.2. Make the volume 100% with WFI and stir the solution for homogeneity. Required amounts of formulation buffer and phenol are mixed to form a homogenous mixture. Calculated amount of rHu FSH drug substance is added with continuous stirring till complete formation of homogenous mixture.

**Table 1: Formulation of hFSH**

| **Sr. No.** | **Ingredient** | **Concentration** |
|---|---|---|
| 1. | Follitropin (hFSH) | 0.05 mg/ml |
| 2. | Trehalose | 30 mg/ml |
| 3. | Mannitol | 30 mg/ml |
| 4. | Sodium Chloride | 5.8 mg/ml |
| 5. | Monobasic Sodium phosphate monohydrate | 0.45 mg/ml |
| 6. | Dibasic Sodium phosphate dihydrate | 1.1 mg/ml |
| 7. | L-Methionine | 0.5 mg/ml |
| 8. | Polysorbate 20 | 0.1 mg/ml |
| 9. | Phenol | 3 mg/ml |
| 10. | Water For Injection | |

### Example 3: Stability test of hFSH aqueous formulations

The biological tests have been performed in compliance with the regulations of the European Pharmacopeia.

### Protein concentration by SE-HPLC

The formulation of Example 1 was evaluated for protein content for FSH using Size exclusion HPLC method.

Protein content was measured at zero time and after 1, 2, 3 and 6 months storage of the formulation at 5±3°C, 25±2°C. The results are listed in Table 1 as micrograms of FSH per gram of solvent.

### Reverse Phase HPLC analysis for oxidation

The aqueous formulation of Example 1 was evaluated for protein content for FSH using a reverse phase HPLC method.

Purity was measured at zero time, and after 1, 2, 3 and 6 months storage of the formulation at 5±3°C, 25±2°C. The results are listed in Table 2.

### Dissociation

For a formulation of as given in Table 1, the percentage of free subunit was evaluated by SDS-PAGE. Measurements were made at zero time, and after 1, 2, 3 and 6 months storage at 5±3°C, 25±2°C. The results are reported in Table 2.

### In vivo assay for FSH

The formulation of Table 1 was tested for FSH activity using the in vivo bioassay at zero time and after 1, 2, 3 and 6 months of storage at 5±3°C, 25±2°C. The results are provided in Table 2 as international units per gram of solvent.

### pH

The pH of the formulation of Table 1 was measured at zero time and after 1, 2, 3 and 6 months storage at 5±3°C, 25±2°C. Results are listed in Table 2.

### Oligomer determination by SE-HPLC

Percentage of oligomer for the formulation of Table 1 was measured at zero time and after 1, 2, 3 and 6 months storage at 5±3°C, 25±2°C by SE-HPLC. Results are provided in Table 2.

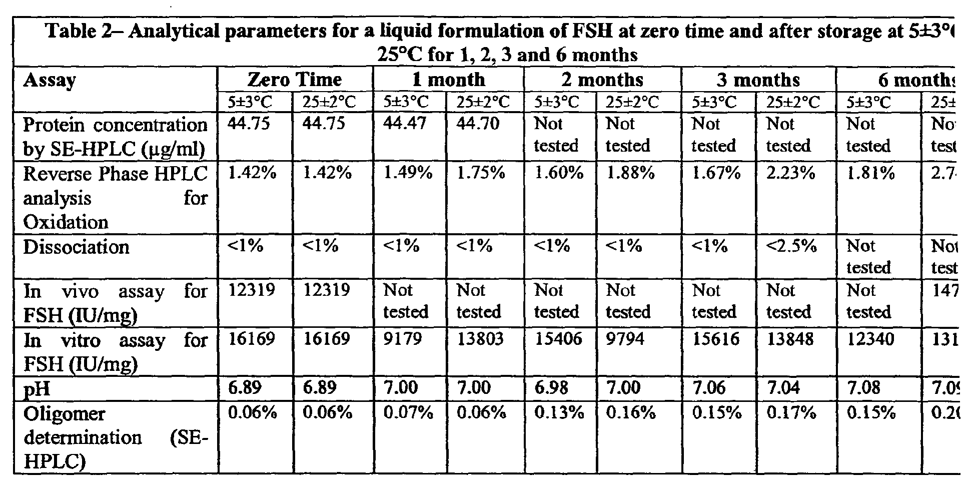

### Example 4: Stability test of hFSH aqueous formulations

### Protein concentration by SE-HPLC

The formulation as provided in Table 1 was evaluated for protein content for FSH using Size exclusion HPLC method.

Protein content was measured at zero time and after 1, 3, 7 and 15 days storage of the formulation at 40°C. The results are listed in Table 3 as micrograms of FSH per gram of solvent.

### Reverse Phase HPLC analysis for purity

The aqueous formulation of Example 1 was evaluated for protein content for FSH using a reverse phase HPLC method.

Purity was measured at zero time and after 1, 3, 7 and 15 days storage of the formulation at 40°C. The results are listed in Table 3.

### Oxidation

The percentage of oxidized form of FSH was measured by a reverse phase HPLC method.

The percentage of oxidized form of FSH was measured at zero time and after 1 3, 7 and 15 days storage of the formulation at 40°C. The results are listed in Table 3.

### Dissociation

For the formulation of Table 1, the percentage of free subunit was evaluated by SDS-PAGE. Measurements were made at zero time and after 1, 3, 7 and 15 days storage of the formulation at 40°C. The results are provided in Table 3.

### In vivo assay for FSH

The formulation of Table 1 was tested for FSH activity using the in vivo bioassay at zero time and after 1, 3, 7 and 15 days storage of the formulation at 40°C. The results are listed in Table 2 as international units per gram of solvent.

### pH

The pH of the formulation of Example 1 was measured at zero time and after 1, 3, 7 and 15 days storage of the formulation at 40°C. Results are listed in Table 3.

**Table 3: Results of stability tests for hFSH formulation**

| **Assay** | **Zero Time** | **1 day** | **3 days** | **7 days** | **15 days** |
|---|---|---|---|---|---|
| | **40°C** | **40°C** | **40°C** | **40°C** | **40°C** |
| Protein concentration by SE-HPLC (µg/ml) | 44.75 | 44.15 | 42.48 | 42.45 | 41.67 |
| Reverse Phase HPLC analysis for purity Oxidation | 1.42% | 1.57% | 1.6% | 1.74% | 2.11% |
| Dissociation | <1% | <2.5% | = 2.5% | <5% | Not tested |
| In vivo assay for FSH | 12319 | Not tested | Not tested | 12055 | Not tested |
| In vitro assay for FSH | 16169 | 9872 | 11163 | 10110 | 9402 |
| pH | 6.89 | 7.00 | 6.99 | 6.99 | 6.99 |
| Oligomer determination (SE-HPLC) | 0.06% | 0.08% | 0.09% | 0.16% | 0.18% |

As apparent from the above results, an aqueous formulation of the present invention (as provided in Table 1) comprising trehalose and mannitol as a stabilizer, polysorbate as a surfactant, methionine as an anti-oxidising agent, and phenol as a preservative maintained at a pH of about 6.7 and 7.2 using phosphate as buffers according to the invention prevents dissociation, oxidation, denaturation and can stably maintain the activity of a rHu FSH for a prolonged period of time.

The formulation prepared by the said invention comprises an effective amount of biologically active human follicle stimulating hormone which can be used in treating infertility in humans. They are preferably used as injectable aqueous solutions.

A novel, thermostable, aqueous formulation of human follicle stimulating hormone described in the present invention has the following advantages:
1. Involves operational simplicity.
2. Involves use of inorganic salts which prevents the dissociation of alpha and beta subunits of human Follicle stimulating hormone.
3. Provides better stability to the aqueous formulation to maintain its activity for a prolonged period of time to guarantee a reasonable shelf-life.
4. Provides better stability to the aqueous formulation at elevated temperatures.

## Claims

1. An aqueous formulation of human follicle stimulating hormone (FSH) comprising sodium chloride as thermostable agent, trehalose and mannitol as stabilizers, polysorbate 20 as non-ionic surfactant, L-methionine as anti-oxidant, phenol as a preservative, and a phosphate buffer providing pH of about 6.5 to 7.2.

2. The formulation of claim 1, which comprises human FSH in a concentration of 0.05 mg/mL, sodium chloride in a concentration of 5.8 mg/mL, trehalose in a concentration of 30 mg/mL, mannitol in a concentration of 30 mg/mL, L-methionine in a concentration of 0.5 mg/mL, Polysorbate 20 in a concentration of 0.1 mg/mL, phenol in a concentration of 3 mg/mL, and a phosphate buffer in a concentration of 1.55 mg/mL providing pH of 7.0 ± 0.2.

## Patentansprüche

1. Wässrige Formulierung von humanem follikelstimulierendem Hormon (FSH), umfassend Natriumchlorid als thermostabiles Mittel, Trehalose und Mannitol als Stabilisatoren, Polysorbat 20 als nichtionisches Tensid, L-Methionin als Antioxidationsmittel, Phenol als Konservierungsstoff und einen Phosphatpuffer, der einen pH-Wert von etwa 6,5 bis 7,2 bereitstellt.

2. Formulierung nach Anspruch 1, welche humanes FSH in einer Konzentration von 0,05 mg/ml, Natriumchlorid in einer Konzentration von 5,8 mg/ml, Trehalose in einer Konzentration von 30 mg/ml, Mannitol in einer Konzentration von 30 mg/ml, L-Methionin in einer Konzentration von 0,5 mg/ml, Polysorbat 20 in einer Konzentration von 0,1 mg/ml, Phenol in einer Konzentration von 3 mg/ml und einen Phosphatpuffer in einer Konzentration von 1,55 mg/ml, der einen pH-Wert von 7,0 ± 0,2 bereitstellt, umfasst.

## Revendications

1. Formulation aqueuse de l'hormone de stimulation folliculaire humaine (FSH) comprenant du chlorure de sodium en tant qu'agent thermostable, du tréhalose et du mannitol en tant que stabilisants, du polysorbate 20 en tant qu'agent tensioactif non ionique, de la L-méthionine en tant qu'antioxydant, du phénol en tant que conservateur et un tampon phosphate assurant un pH d'environ 6,5 à 7,2.

2. Formulation selon la revendication 1, qui comprend de la FSH humaine dans une concentration de 0,05 mg/ml, du chlorure de sodium dans une concentration de 5,8 mg/ml, du tréhalose dans une concentration de 30 mg/ml, du mannitol dans une concentration de 30 mg/ml, de la L-méthionine dans une concentration de 0,5 mg/ml, du polysorbate 20 dans une concentration de 0,1 mg/ml, du phénol dans une concentration de 3 mg/ml, et un tampon phosphate dans une concentration de 1,55 mg/ml assurant un pH de 7,0 ± 0,2.
